# EUROPÄISCHE PATENTSCHRIFT

(11) **EP 0 490 107 B1**
(45) Veröffentlichungstag und Bekanntmachung des Hinweises auf die Patenterteilung: **01.03.1995**
(21) Anmeldenummer: 91119356.3
(22) Anmeldetag: 13.11.1991
(51) Int. Cl.: A61B 6/04

(54) **Patientenuntersuchungstisch**
Examination table for patients
Table d'examen pour patient

(30) Priorität: 12.12.1990 SE 9003968
(43) Veröffentlichungstag der Anmeldung: 17.06.1992
(73) Patentinhaber: Siemens-Elema AB, 171 95 Solna 1 (SE); SIEMENS AKTIENGESELLSCHAFT, 80333 München (DE)
(72) Erfinder: Larsson, Sten, S-162 40 Vällingby (SE)

(56) Entgegenhaltungen:
- CH-A- 476 489
- DE-C- 3 222 332
- US-A- 4 894 855

## Beschreibung

Die Erfindung betrifft einen Patientenuntersuchungstisch, der an seiner einen Stirnseite an einem Decken- oder Bodenstativ über eine Kippvorrichtung befestigt ist, die ein Kippen des Untersuchungstisches nach oben oder nach unten in dessen Längsrichtung erlaubt, wie es aus der US-A-4 894 855 bekanntgeworden ist.

Ein Patientenuntersuchungstisch dieser Art, der bei Röntgenuntersuchungen verwendet wird, ist durch den Siemens-Prospekt "KOORDINAT 3D II" bekannt. Der Untersuchungstisch, der um die eine Stirnseite kippbar ist, ist an einem Deckenstativ befestigt, durch das der Untersuchungstisch auch seitlich, in der Höhe und in seiner Längsrichtung verschiebbar ist. Bei einigen Untersuchungen besteht der Bedarf, den Tisch während der Untersuchung zu kippen. Wenn eine Röntgenuntersuchung im Bereich des Bauches erfolgt, muß das Stativ für die Röntgenröhre und der Bildverstärker sowie der Untersuchungstisch umpositioniert werden, um eine Kippung des Tisches um den Bauch des Patienten Zu ermöglichen, da der Tisch sonst entweder mit der Röntgenröhre oder mit dem Bildverstärker zusammenstoßen würde. Die Kippung des Tisches erfolgt manuell.

Ein weiterer Röntgenuntersuchungstisch ist durch durch den Siemens-Prospekt "CIRRUS" bekannt. Auch dieser Untersuchungstisch ist an einem Deckenstativ befestigt, was eine Längs-, Seiten- und Höhenverschiebung des Tisches ermöglicht. Der Untersuchungstisch ist außerdem um eine Welle drehbar, die direkt unterhalb des Tisches angeordnet aber gegenüber dessen Mitte verschoben ist. Der Untersuchungstisch ist außerdem mit einer elektrischen Regelvorrichtung mit einer Folgeregelung versehen, durch die der Tisch derart steuerbar ist, daß sich der zu untersuchende Bereich immer in einem Isozentrum befindet. Bei einer Röntgenuntersuchung der Brust oder des Bauches kann der Untersuchungstisch mit Hilfe der Folgeregelung um diesen Bereich gekippt werden ohne daß die Gefahr besteht, daß der Patient oder der Tisch mit der Röntgenröhre oder mit dem Bildverstärker kollidiert.

Der Erfindung liegt die Aufgabe zugrunde einen Untersuchungstisch der eingangs genannten Art zu schaffen, der mit verhältnismäßig einfachen und daher billigen Mitteln eine Kippung um eine fiktive Achse ermöglicht, die in einem fiktiven Raum etwa in der Mitte des Untersuchungstisches bewegbar ist.

Diese Aufgabe ist erfindungsgemäß dadurch gelöst, daß die Kippvorrichtung ein erstes Zahnrad umfaßt, das am Stativ fest angebracht ist, ein zweites Zahnrad aufweist, das am Untersuchungstisch fest angebracht und um die Welle des ersten Zahnrades schwenkbar ist, sowie eine Transmission zwischen dem ersten und dem zweiten Zahnrad aufweist, so daß, wenn die Kippvorrichtung um die Welle des ersten Zahnrades in die eine Richtung gedreht wird, das zweite Zahnrad in der entgegengesetzten Richtung gedreht wird. Hierdurch ist eine in dem Aufbau sehr einfache Kippvorrichtung gegeben, die es ermöglicht, daß der Untersuchungstisch in seinem mittleren Bereich gekippt werden kann. Dadurch, daß die Kippvorrichtung im Bereich der einen Stirnseite des Untersuchungstisches befestigt ist, sind im Kippbereich des Tisches keine mechanischen Teile vorhanden, wodurch eine sehr gute Erreichbarkeit für das Personal und die Röntgenausrüstung gegeben ist.

Im Hinblick auf eine günstige konstruktive Ausgestaltung der Erfindung wird vorgeschlagen, daß die Kippvorrichtung eine ungerade Anzahl von mindestens drei Zahnräder beinhaltet, die ineinandergreifen. Hierdurch ist der Aufbau der Kippvorrichtung sehr stabil.

In einer weiteren einfachen Ausführungsform der Erfindung wird vorgeschlagen, daß mindestens ein Arm vorgesehen ist, der parallel und entlang der Zahnräder verläuft und an dem die Zahnräder angebracht sind.

In einer vorteilhaften Ausbildung der Erfindung wird vorgeschlagen, daß auf jeder Seite der Zahnräder ein Arm angebracht ist, von denen der eine Arm ein Gehäuse für die Kippvorrichtung und der andere Arm einen Deckel für das Gehäuse bildet. Durch diese Ausführungsform wird die Zahl der Teile für die Kippvorrichtung verringert.

In einer Weiterbildung der Erfindung wird vorgeschlagen, daß mindestens der eine Arm mit einer Antriebsvorrichtung verbunden ist, mittels derer der Arm um die Welle des ersten Zahnrades schwenkbar ist. Davon ausgehend wird in einer vorteilhaften Weiterbildung vorgeschlagen, daß die Antriebsvorrichtung ein Antriebsrad umfaßt, dessen Welle mit der Welle des ersten Zahnrades zusammenfällt, wobei das Antriebsrad mit einem Antriebsmotor verbunden ist. Hierdurch ist eine in der Ausführung einfache Steuerung der Kippung des Untersuchungstisches gegeben.

Weitere Vorteile und Einzelheiten ergeben sich aus den Unteransprüchen.

Die Erfindung ist nachfolgend anhand eines in der Zeichnung dargestellten Ausführungsbeispiels näher erläutet. Es zeigen:
- FIG. 1: eine Seitenansicht eines Untersuchungstisches nach der Erfindung,
- FIG. 2: eine Draufsicht des Untersuchungstisches nach FIG. 1
- FIG. 3 und 4: Seitenansichten des Untersuchungstisches nach FIG 1 und 2 in verschiedenen Lagen gekippt und
- FIG. 5: schematisch das Funktionsprinzip der Kippvorrichtung des Untersuchungstisches nach der Erfindung.

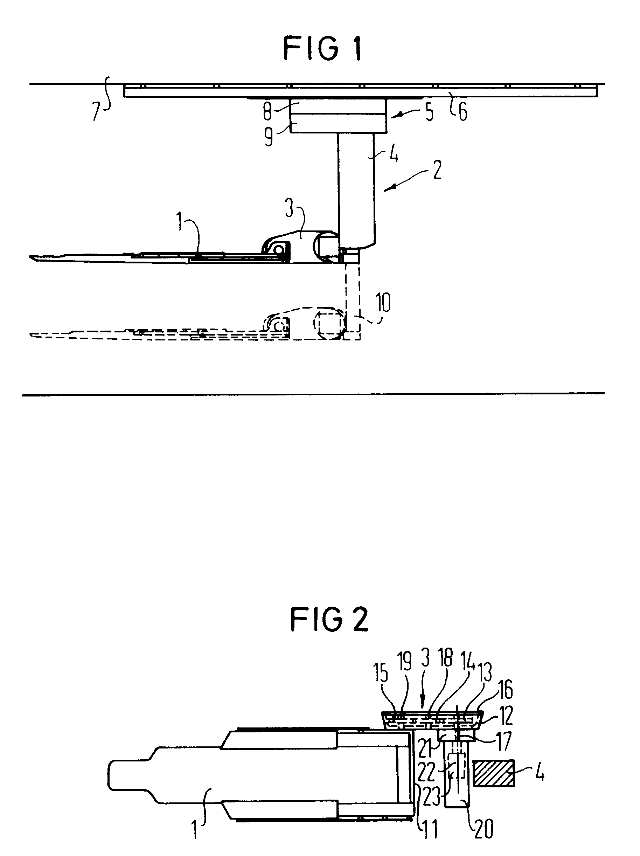

In FIG. 1 ist ein Untersuchungstisch 1 gezeigt, der an seiner einen Stirnseite über eine Kippvorrichtung 3 an einem Deckenstativ 2 befestigt ist. Das Deckenstativ 2 ist mit einer Säule 4 versehen, die an einer Halterung 5 befestigt ist, welche in an der Decke 7 befestigten Schienen 6 läuft. Die Halterung 5 ist ihrerseits in ein oberes Teil 8 und ein unteres Teil 9 unterteilt, wobei das untere Teil 9 gegenüber dem oberen Teil 8 senkrecht zur Längsrichtung der Deckenschienen 6 verschiebbar ist. Die Säule 4 ist außerdem mit einem Arm 10 versehen, der die Säule 4 nach unten verlängern kann. Durch das Deckenstativ 2 ist eine Verschiebung des Untersuchungstisches 1 in der Länge, Quere und in der Höhe möglich.

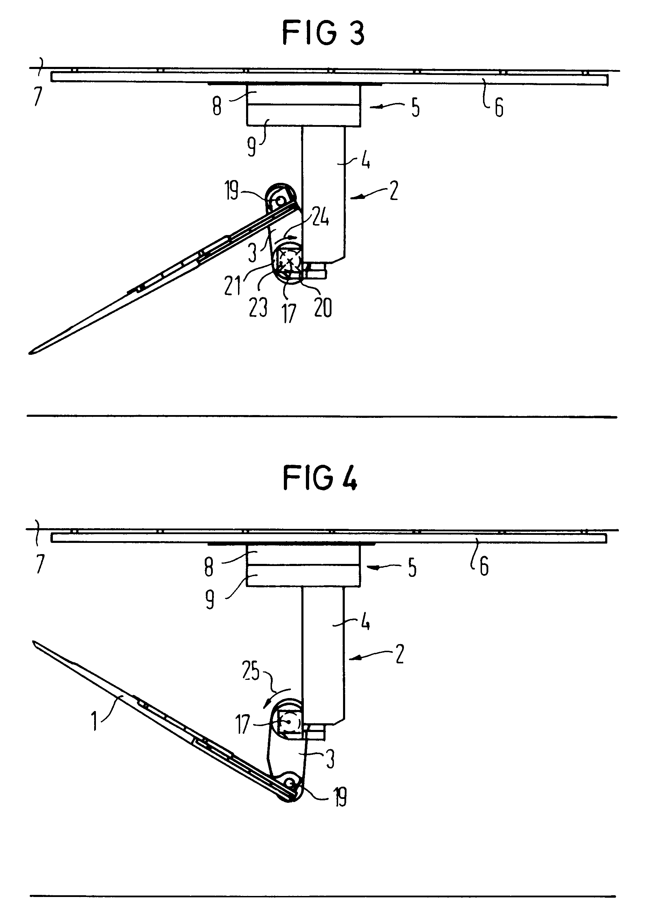

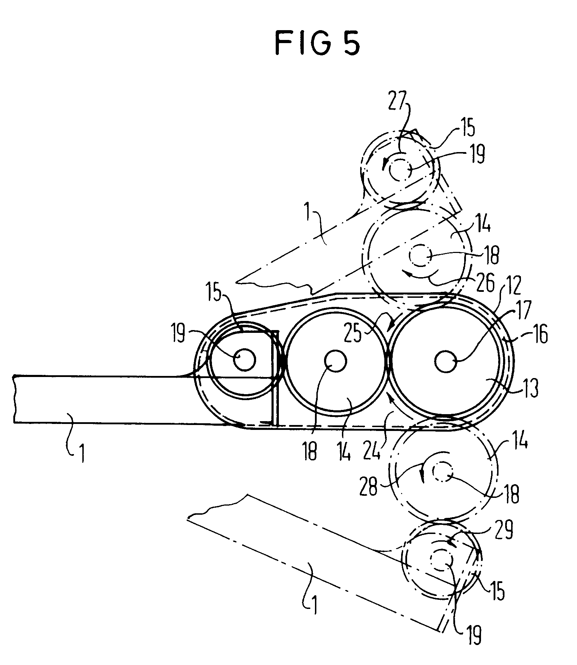

In der FIG. 2 ist gezeigt, daß der Untersuchungstisch 1 an der Kippvorrichtung 3 über ein L-förmiges Halteteil 11 befestigt ist. In dieser FIG. ist auch dargestellt, daß die Kippvorrichtung 3 ein Gehäuse 12, in dem Zahnräder 13,14,15 angeordnet sind, und einen Deckel 16 für das Gehäuse 12 aufweist. Die Zahnräder 13,14,15 sind über Achsen bzw. Wellen 17,18,19 teils mit dem Gehäuse 12 und teils mit dem Deckel 16 verbunden. Das Zahnrad 13 ist außerdem über die Welle 17 an einem an der Säule 4 befestigten Ausleger 20 fest angebracht, der die Kippvorrichtung 3 und den Untersuchungstisch 1 trägt. Das Zahnrad 15 ist dagegen an dem Untersuchungstisch 1 über die Drehachse 19 fest angeordnet. Das Gehäuse 12 ist mit einer Antriebsvorrichtung fest verbunden, die ein mit einem Antriebsmotor 23 verbundenes Antriebsrad 21 aufweist, dessen Welle 22 mit der Welle 17 des Zahnrades 13 zusammenfällt. Durch Drehen des Antriebsrades 21 mit Hilfe des Antriebmotors 23 in Richtung des Pfeiles 24, was in FIG 3 gezeigt ist, wird auch die Kippvorrichtung 3 um die Welle 17 in derselben Richtung gedreht. Hierdurch wird der Untersuchungstisch 1 durch die Kippvorrichtung 3, deren Funktion in Verbindung mit der FIG. 5 beschrieben wird, in der entgegengesetzten Richtung gedreht, so daß eine Kippung des Untersuchungstisches 1 nach unten um eine fiktive Achse erfolgt, die sich während des Kippens in einem fiktiven Raum etwa in der Mitte des Untersuchungstisches 1 und quer zu dessen Längsrichtung bewegt.

In der FIG. 4 ist dargestellt, daß der Untersuchungstisch 1 auch in eine nach oben gerichtete Lage gekippt werden kann. Dies erfolgt, indem das Antriebsrad 21 mit Hilfe des Antriebsmotors 23 die Kippvorrichtung 3 um die Welle 17 in Richtung des Pfeiles 25 dreht. Gleichzeitig wird der Untersuchungstisch 1 in der entgegengesetzten Richtung gedreht, so daß eine Kippung um die genannte fiktive Achse erfolgt.

In der FIG. 5 ist die Kippvorrichtung 3 schematisch gezeigt. In dieser FIG. ist dargestellt, daß die Zahnräder 13,14,15, die an dem Gehäuse 12 und dem Deckel 16 angebracht sind, ineinandergreifen. Wie bereits beschrieben, ist das Zahnrad 13 durch die Welle 17 fest am Deckenstativ 2 angebracht und das Zahnrad 15 über die Drehachse 9 am Untersuchungstisch 1 befestigt. Das Zahnrad 14 ist dagegen über die Achse 18 an dem Gehäuse 12 bzw. an dem Deckel 16 drehbar angeordnet. Wenn das Antriebsrad 21, wie es in FIG. 2 gezeigt ist, mit Hilfe des Antriebsmotors 23 das Gehäuse 12 bzw. den Deckel 16 dazu zwingt, sich um die Welle 17 in Richtung des Pfeiles 24 zu drehen, dreht sich das Zahnrad 14 um seine Achse 18 in die gleiche Richtung, wie es der Pfeil 26 zeigt. So wird das Zahnrad 15 gezwungen sich in die entgegengesetzte Richtung zu drehen, wie es der Pfeil 27 zeigt, wobei der Untersuchungstisch 1 nach und nach um die Drehachse 19 in eine Position gekippt wird, wie sie in FIG. 3 gezeigt ist. Wenn die Kippvorrichtung 3 um die Welle 17 in Richtung des Pfeiles 25 gedreht wird, wird das Zahnrad 14 um seine Achse 18 in die gleiche Richtung gedreht, wie es der Pfeil 28 zeigt. Auf diese Weise wird das Zahnrad 15 gezwungen sich in die entgegengesetzte Richtung zu drehen, wie es der Pfeil 29 zeigt; gleichzeitig wird der Untersuchungstisch 1 durch die Bewegung des Zahnrades 15 nach und nach um die Drehachse 19 in eine Lage gekippt, wie sie in FIG. 4 gezeigt ist.

Der Kippwinkel des Untersuchungstisches 1 ist von der Übersetzung zwischen den Zahnrädern 13 und 15 abhängig. Die Übersetzung zwischen diesen Zahnrädern 13 und 15 kann selbstverständlich auch so gewählt werden, daß weitere gewünschte Kippwinkel erreicht werden können. Auf diese Weise kann die fiktive Achse, um die der Untersuchungstisch gekippt wird, verschoben werden.

In einer weiteren Ausführungsform der Kippvorrichtung kann das Zahnrad 14 durch eine Ketten- och Riementransmission ersetzt werden, die die verbleibenden Zahnräder 13 und 15 miteinander verbindet. Bei einer derartigen Transmission zwischen den Zahnrädern 13 und 15 ist es möglich, das Gehäuse 12 und den Deckel 16, die der Kreisbewegung des Zahnrades 15 um die Welle 17 folgen, durch zwei einander gegenüberliegende parallele Platten zu ersetzen, zwischen denen das Getriebe angeordnet ist, und die mit bogenförmigen Schlitzen versehen sind, in denen die Drehachse 19 des Zahnrades 15 laufen kann. Bei einem derartigen Aufbau würde die Bewegungen des Zahnrades 15 entlang des bogenförmigen Schlitzes vorteilhaft mittels einer Antriebsvorrichtung gesteuert werden.

## Patentansprüche

1. Patientenuntersuchungstisch, der an seiner einen Stirnseite an einem Decken- oder Bodenstativ über eine Kippvorrichtung befestigt ist, die ein Kippen des Untersuchungstisches nach oben oder nach unten in dessen Längsrichtung erlaubt, **dadurch gekennzeichnet**, daß die Kippvorrichtung ein erstes Zahnrad (13) umfaßt, das am Stativ (2) fest angebracht ist, ein zweites Zahnrad (15) aufweist, das am Untersuchungstisch (1) fest angebracht und um die Welle (17) des ersten Zahnrades (13) schwenkbar ist, sowie eine Transmission zwischen dem ersten und dem zweiten Zahnrad (13,15) aufweist, so daß, wenn die Kippvorrichtung (3) um die Welle (17) des ersten Zahnrades (13) in die eine Richtung gedreht wird, das zweite Zahnrad (15) in der entgegengesetzten Richtung gedreht wird.

2. Patientenuntersuchungstisch nach Anspruch 1, **dadurch gekennzeichnet,** daß die Kippvorrichtung (3) eine ungerade Anzahl von mindestens drei Zahnrädern (13,13,15) beinhaltet, die ineinandergreifen.

3. Patientenuntersuchungstisch nach Anspruch 1, **dadurch gekennzeichnet,** daß als Transmission eine Kette vorgesehen ist.

4. Patientenuntersuchungstisch nach Anspruch 1, **dadurch gekennzeichnet,** daß als Transmission ein Antriebsriemen vorgesehen ist.

5. Patientenuntersuchungstisch nach einem der Ansprüche 1 bis 4, **dadurch gekennzeichnet**, daß mindestens ein Arm (12,16) vorgesehen ist, der parallel und entlang der Zahnräder (13,14,15) verläuft und an dem die Zahnräder (13,14,15) angebracht sind.

6. Patientenuntersuchungstisch nach einem der Ansprüche 1 bis 5, **dadurch gekennzeichnet**, daß auf jeder Seite der Zahnräder (13,14,15) jeweils ein Arm (12,16) angebracht ist, von denen der eine Arm (12) ein Gehäuse für die Kippvorrichtung (3) und der andere Arm (16) einen Deckel für das Gehäuse bildet.

7. Patientenuntersuchungstisch nach einem der Ansprüche 1 bis 6, **dadurch gekennzeichnet**, daß mindestens der eine Arm (12) mit einer Antriebsvorrichtung (21,23) verbunden ist, mittels derer der Arm (12) um die Welle (17) des ersten Zahnrades (13) schwenkbar ist.

8. Patientenuntersuchungstisch nach Anspruch 7, **dadurch gekennzeichnet**, daß die Antriebsvorrichtung (21,23) ein Antriebsrad (21) umfaßt, dessen Welle (22) mit der Welle (17) des ersten Zahnrades (13) zusammenfällt, wobei das Antriebsrad (21) mit einem Antriebsmotor (23) verbunden ist.

## Claims

1. Patient-examination table, which is fastened at its one end face to a ceiling or floor stand via a tilting device enabling the examination table to be tilted upwards or downwards in its longitudinal direction, characterized in that the tilting device comprises a first gearwheel (13), which is fixed to the stand (2), exhibits a second gearwheel (15), which is fixed to the examination table (1) and is pivotable about the shaft (17) of the first gearwheel (13), and exhibits a transmission between the first and the second gearwheel (13, 15), so that, when the tilting device (3) is rotated about the shaft (17) of the first gearwheel (13) in the one direction, the second gearwheel (15) is rotated in the opposite direction.

2. Patient-examination table according to Claim 1, characterized in that the tilting device (3) contains an odd number of at least three gearwheels (13, 14, 15), which intermesh.

3. Patient-examination table according to Claim 1, characterized in that, as the transmission, a chain is provided.

4. Patient-examination table according to Claim 1, characterized in that, as the transmission, a drive belt is provided.

5. Patient-examination table according to one of Claims 1 to 4, characterized in that at least one arm (12, 16) is provided, which runs parallel to and along the gearwheels (13, 14, 15) and to which arm (12, 16) the gearwheels (13, 14, 15) are fitted.

6. Patient-examination table according to one of Claims 1 to 5, characterized in that on each side of the gearwheels (13, 14, 15) an arm (12, 16) is respectively fitted, the one arm (12) of which gearwheels (13, 14, 15) forms a housing for the tilting device (3) and the other arm (16) forms a lid for the housing.

7. Patient-examination table according to one of Claims 1 to 6, characterized in that at least the one arm (12) is connected to a drive device (21, 23), by means of which the arm (12) is pivotable about the shaft (17) of the first gearwheel (13).

8. Patient-examination table according to Claim 7, characterized in that the drive device (21, 23) comprises a drive wheel (21), the shaft (22) of which coincides with the shaft (17) of the first gearwheel (13), the drive wheel (21) being connected to a drive motor (23).

## Revendications

1. Table d'examen pour patient, qui est fixée, par l'une de ses faces frontales, à un statif plafonnier ou à un statif placé au sol par l'intermédiaire d'un dispositif de basculement, qui permet un basculement de la table d'examen vers le haut et vers le bas, dans sa direction longitudinale, caractérisée par le fait que le dispositif de basculement comprend un premier pignon (13), qui est monté fixe sur le statif (2), un second pignon (15) qui est monté fixe sur la table d'examen et peut basculer autour de l'arbre (17) du premier pignon (13), ainsi qu'une transmission disposée entre les premier et second pignons (13,15), de sorte que, lorsque le dispositif de basculement (3) tourne dans un sens autour de l'arbre (17) du premier pignon (13), le second pignon (15) tourne en sens opposé.

2. Table d'examen pour patient suivant la revendication 1, caractérisée par le fait que le dispositif de basculement (3) comporte un nombre impair, égal au moins à trois, de pignons (13,14,15), qui engrènent les uns dans les autres.

3. Table d'examen pour patient suivant la revendication 1, caractérisée par le fait qu'une chaîne est prévue comme transmission.

4. Table d'examen pour patient suivant la revendication 1, caractérisée par le fait qu'une courroie d'entraînement est prévue comme transmission.

5. Table d'examen pour patient suivant l'une des revendications 1 à 4, caractérisée par le fait qu'il est prévu au moins un bras (12, 16), qui s'étend parallèlement aux et le long des pignons (13,14,15) et sur lequel sont montés ces derniers.

6. Table d'examen pour patient suivant l'une des revendications 1 à 5, caractérisée par le fait que de chaque côté des pignons (13,14,15) sont disposés respectivement des bras (12,16), dont l'un (12) forme un boîtier pour le dispositif de basculement (3) et dont l'autre (16) forme un couvercle pour le boîtier.

7. Table d'examen pour patient suivant l'une des revendications 1 à 6, caractérisée par le fait qu'au moins l'un des bras (12) est relié à un dispositif d'entraînement (21,23), au moyen duquel le bras (12) peut basculer autour de l'arbre (17) du premier pignon (13).

8. Table d'examen pour patient suivant la revendication 7, caractérisée par le fait que le dispositif d'entraînement (21,23) comprend une roue d'entraînement (21), dont l'arbre (22) est coaxial à l'arbre (17) du premier pignon (13), la roue d'entraînement (21) étant reliée à un moteur d'entraînement (23).
